# EUROPÄISCHE PATENTSCHRIFT

(11) **EP 0 583 711 B1**
(45) Veröffentlichungstag und Bekanntmachung des Hinweises auf die Patenterteilung: **05.11.1997**
(21) Anmeldenummer: 93112682.5
(22) Anmeldetag: 07.08.1993
(51) Int. Cl.: A61F 2/36, A61F 2/40

(54) **Kugelkopfendoprothese**
Ball headed endoprosthesis
Endoprothèse à tête sphérique

(30) Priorität: 17.08.1992 DE 4227139
(43) Veröffentlichungstag der Anmeldung: 23.02.1994
(73) Patentinhaber: ESKA Implants GmbH & Co., 23556 Lübeck (DE)
(72) Erfinder: Grundei, Hans, D-23558 Lübeck (DE); Moser, Heinz, D-65375 Oestrich-Winkel (DE)
(74) Vertreter: Fuchs Mehler Weiss

(56) Entgegenhaltungen:
- EP-A- 0 363 019
- CH-A- 507 704
- DE-A- 2 921 529
- DE-U- 8 901 018
- DE-U- 9 211 250
- FR-A- 2 574 283
- FR-A- 2 619 502
- US-A- 3 685 058

## Beschreibung

Die Erfindung betrifft eine Kugelgelenkendoprothese mit den Merkmalen des Oberbegriffs des Anspruchs 1, wie sie beispielsweise aus der EP-A-0 363 019 bekannt ist.

Derartige Kugelgelenkendoprothesen, beispielsweise in Ausbildung einer Total-Hüftgelenksendoprothese, ermöglichen nach dem Einbringen des Stiels in den Medularraum des Femurknochen die exakte Orientierung des Stielteils dahingehend zu erzielen, daß eine mit dem Femurstielteil zu verbindende künstliche Gelenkkugel nach der Implantation keinerlei Luxationstendenzen aufweist. Eine Anpassung an die individuellen Patientenerfordernisse umfaßt daneben gegebenenfalls eine Beinverlängerung, ein kräftemäßiger Abgleich der muskulären Antagonisten in allen vier Richtungen (medial, lateral; dorsal, ventral), die Kompensation einer Valgus- oder Varusstellung des betreffenden Beines.

Es ist bei dieser gattungsgemäßen Endoprothese vorgesehen, daß die Achse der konischen Bohrung mit der Durchmesserlinie, die durch den Pol und den geometrischen Mittelpunkt des Kugelkopfes geht, einen Winkel im Bereich ca. von 7° bis 15° einschließt. Die konische Bohrung ist also in dem besagten Winkelbereich sozusagen schief in den Kugelkopf eingebracht. Darüber hinaus ist sie im Kugelkopf mit einem Versatz von 1 bis 4 mm gegenüber der Durchmesserlinie dezentriert angeordnet. Zu dem winkligen Versatz zwischen 7° und 15° tritt also ein translatorischer Versatz von 1 bis 4 mm. Wird dieser Kugelkopf auf den konischen Zapfen gesetzt und gedreht, so ist einsichtig, daß der geometrische Mittelpunkt des Kugelkopfes im Abstand zwischen 1 bis 4 mm (je nach Betrag des Versatzes) um die Längsachse des konischen Zapfen versetzt gedreht wird. Gleichzeitig beschreibt der Pol des Kugelkopfes eine Ortskurve in Form eines Kreises. Dabei schließen die Längsachse des konischen Zapfens und die Durchmesserlinie durch den Pol und den geometrischen Mittelpunkt des Kugelkopfes einen Winkel zwischen ± 7° und ± 15° ein, je nachdem mit welchem Winkel die konische Bohrung in den Kugelkopf eingebracht ist, wobei die Bezugslinie die Längsachse des konischen Zapfens ist. Aus dem vorstehenden wird deutlich, welchen Freiheitsgrad der Operateur während der Operation hat, um die für den jeweiligen Patienten optimale Stellung des Kugelkopfes in bezug auf das Basisteil zu finden. Hierdurch lassen sich in situ die bereits angesprochenen Einstellungen problemlos vornehmen, also insbesondere eine Valgus- oder Varusstellung des Schenkelhalses des betreffenden Beines kompensieren, die Länge des Beines verändern und die Spannung der muskulären Antagonisten medial gegen lateral und dorsal gegen ventral vergleichmäßigen.

Allen diesen Kugelgelenksendoprothesen ist gemeinsam, daß der Kugelkopf in bezug auf den konischen Steckzapfen verstellt werden kann.

Vor diesem Hintergrund ist es nun die Aufgabe der vorliegenden Erfindung, eine Kugelkopfendoprothese der eingangs genannten Art so weiter zu bilden, daß die einmal als optimal befundene Stellung des Kugelkopfes in bezug auf das Basisteil problemlos in situ wieder aufgefunden werden kann und daß die Stabilität der gewählten Kugelkopfstellung in bezug auf das Basisteil gewährleistet wird.

Gelöst wird diese Aufgabe durch das kennzeichnende Merkmal des Anspruchs 1. Vorteilhafte Weiterbildungen ergeben sich aus den Unteransprüchen.

Im Inneren der konischen Bohrung im Kugelkopf sind demnach mindestens fünf achsparallele Nuten vorgesehen, in die nach Herstellung der konischen Klemmverbindung mit dem konischen Zapfen des Basisteils eine an der Umfangsfläche des konischen Zapfens vorgesehene Arretierungnase greift. Die mindestens fünf achsparallelen Nuten sind äquidistant auf der Innenseite der Bohrung verteilt. Im Falle von fünf Nuten sind die Nuten also in einem Winkel von 72° angulär beabstandet.

Die erfindungsgemäße Lösung läßt sich nicht realisieren durch die Ausbildung des Zapfens als Keilwelle, wie es in der bekanntgewordenen DE-A-29 21 529 vorgesehen ist. Abgesehen davon, daß die darin gezeigte Keilwelle eine zylindrische Mantelfläche aufweist, ist sie symmetrisch aufgebaut, so daß der Operateur keinerlei Anhaltspunkte hat, in welche Stellung der Kugelkopf aufgebracht werden kann, um die einmal als optimal empfundene Stellung des Kugelkopfes auf dem Basisteil wieder zu erlangen. Aufgrund der Symmetrie erscheint dem Operateur jede mögliche Stellung als gleichwertig, ohne daß er schildern könnte, wie der Kugelkopf auf dem Basisteil gesessen hat, um die medizinisch einwandfreie Lage einzunehmen.

Die erwähnte Arretierungsnase ist am proximalen Ende des konischen Zapfens, also an dem dem Kugelkopf zugewandten Ende vorgesehen, und zwar in medialer Richtung weisend. Dies erhöht die Bruchsicherheit der Arretierungsnase und damit die Stabilität der gewählten Kugelkopfstellung in bezug auf das Basisteil erheblich. Damit nämlich befindet sich die Arretierungsnase auf der am wenigsten belasteten Seite des konischen Zapfens. Gleichwohl ist der Eingriff in eine der Nuten in dem Inneren der konischen Bohrung des Kugelkopfes auch bei unterschiedlich großen Kugelköpfen gesichert. Damit kann dem Operateur eine Art Bausatz standardisierter Endoprothesenteile zur Verfügung gestellt werden, bestehend aus standardisierten Basisteilen und standardisierten Kugelköpfen, beispielsweise unterschiedlicher Größe und mit unterschiedlichen winkligen Versatz der konischen Bohrung im Kugelkopf und/oder mit unterschiedlicher Exzentrität der konischen Bohrung im Kugelkopf, so daß der Operateur während der Operation die optimal passende Kugelgelenkendoprothese zusammenstellen kann.

Gemäß einer besonders bevorzugten Ausführungsform sind auf der Innenseite der konischen Bohrung zwölf achsparallele und äquidistante Nuten vorgesehen, die jeweils einen angulären Abstand von 30° zueinander aufweisen. Hierdurch wird eine im allgemeinen ausreichende Variabilität gewährleistet.

Die Erfindung wird anhand der Zeichnungen beispielhaft näher erläutert. Hierbei zeigt:
- Fig. 1: die Seitenansicht eines Basisteils, hier eines Femurstielteils,
- Fig. 2: eine Schnittansicht des Kugelkopfes,
- Fig. 3: die Ansicht des Kugelkopfes aus Fig. 2 in Richtung des Pfeils III,
- Fig. 4: die Ansicht auf den konischen Zapfen des Basisteils gemäß Fig. 1 in Richtung des Pfeils IV,

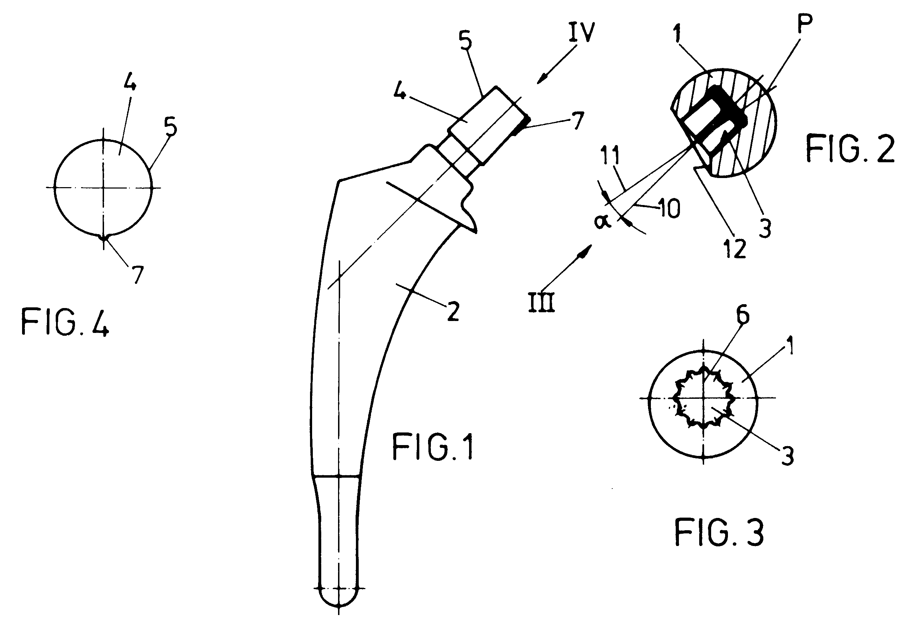

- Fig. 4: eine Seitenansicht, teilweise im Schnitt, der zusammengesetzten Kugelkopfendoprothese in einer Valgusstellung und

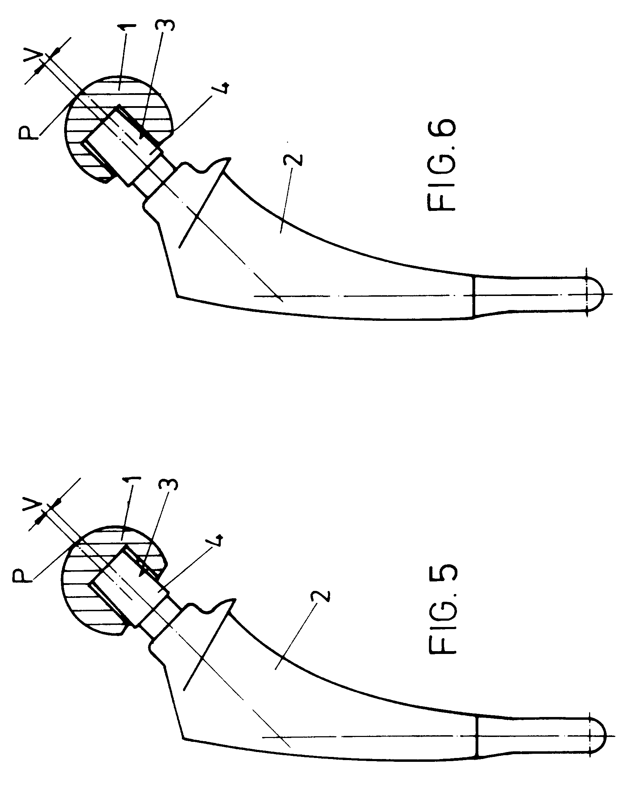

- Fig. 5: eine entsprechende Seitenansicht wie Fig. 4, jedoch in Varusstellung.

Nachfolgend sind dieselben Teile mit gleichen Bezugszeichen versehen.

In Figur 1 ist dargestellt ein Basisteil 2, hier als Femurstielteil ausgebildet. An dessen gelenkseitigem Ende ist ein konischer Zapfen 4 ausgebildet, der zur konischen Klemmverbindung mit dem Kugelkopf 1 vorgesehen ist. Auf der Umfangsfläche 5 des konischen Zapfens 4 ist vorliegend am proximalen Ende des Zapfens 4 eine Arretierungsnase 7 vorgesehen. Diese weist nach medial. Dies hat den Vorteil, daß die Arretierungsnase unter Belastung des Implantats auf der am wenigstens belasteten Seite liegt.

In Figur 2 ist die Schnittansicht des Kugelkopfes 1 der Kugelkopfendoprothese abgebildet. Im Inneren des Kugelkopfes ist eine konische Bohrung 3 vorgesehen, die so dimensioniert ist, daß der Kugelkopf 1 auf dem Basisteil 2 mittels einer konischen Klemmverbindung zwischen der konischen Bohrung 3 und dem konischen Zapfen 4 sitzt.

Wie aus Fig. 2 erkennbar, schließt die Achse 10 der konischen Bohrung 3 mit der Durchmesserlinie 11, die durch den Pol P des Kugelkopfes 1 sowie durch dessen geometrischen Mittelpunkt geht, einen Winkel α ein, der im Bereich zwischen 7° und 15° liegt. Durch die Schrägstellung der Bohrungsachse 10 entsteht in einem Bereich der Bohrungskante eine Fase 12.

Die Bohrung 3 ist aber nicht nur schräg in den Kugelkopf 1 gebohrt. Sie ist von der Durchmesserlinie 11 um einen Versatz V (Figuren 5 und 6) dezentriert. Der Versatzbau kann 1 bis 4 mm betragen.

Fig. 3 zeigt die Ansicht des Kugelkofpes 1 in Richtung des Pfeiles III in Fig. 2. Nochmals deutlich erkennbar ist der translatorische Versatz, also die Dezentrierung der Bohrung 3 im Kugelkopf 1.

Auf dem Innenumfang der Bohrung 3 sind im dargestellten Ausführungsbeispiel zwölf achsparallel verlaufende Nuten 6 vorgesehen. Nach Herstellung der konischen Klemmverbindung zwischen der konischen Bohrung 3 und dem konischen Zapfen 4 greift die Arretierungsnase 7 in eine der Nuten 6 und bildet so eine Rotationssperre gegen ein unbeabsichtigtes Verdrehen unter Belastung der Endoprothese.

Fig. 4 ist eine vergrößerte Ansicht auf den Zapfen 4 in Richtung des Pfeils IV in Fig. 1. Deutlich erkennbar ist die auf der Umfangsfläche 5 des Zapfens 4 sitzende Arretierungsnase 7.

In den Figuren 5 und 6 ist die zusammengesetzte Kugelkopfendoprothese in Form des Femurteils einer Total-Gelenksendoprothese dargestellt. In Figur 5 ist der Kugelkopf in eine solche Stellung gedreht, die beispielsweise zur Kompensation einer Valgusstellung des Beines dient. Die andere Extremlage in dieser Ebene zeigt Fig. 6, bei der also der Kugelkopf 1 gegenüber der Stellung in Fig. 5 um 180° gedreht worden ist, so daß diese Stellung für die Kompensation einer Varusstellung des Beines günstig ist.

## Patentansprüche

1. Kugelkopfendoprothese, bestehend aus einem die natürliche Gelenkkugel nachbildenden Kugelkopf, der mittels einer konischen Klemmverbindung mit einem in einem Knochen zu verankernden Basisteil verbindbar ist, wofür das Basisteil einen konischen Zapfen und der Kugelkopf eine konische Bohrung aufweist, wobei die Achse (10) der konischen Bohrung (3) mit der Durchmesserlinie (11) des Kugelkopfes (1) einen Winkel α im Bereich von 7° < α < 15° einschließt und die konische Bohrung (3) in dem Kugelkopf (1) mit einem Versatz (V) von 1-4 mm gegenüber der Durchmesserlinie (11) dezentriert angeordnet ist, dadurch gekennzeichnet, daß im Inneren der Bohrung (3) mindestens fünf achsparallele Nuten (6) vorgesehen sind, in die nach Herstellung der konischen Klemmverbindung eine an der Umfangsfläche (5) des konischen Zapfens (4) vorgesehene Arretierungsnase (7) greift, die am proximalen Ende des konischen Zapfens (4) in medialer Richtung weisend vorgesehen ist.

2. Kugelkopfendoprothese nach Anspruch 1, dadurch gekennzeichnet, daß auf der Innenseite der konischen Bohrung (3) zwölf achsparallele und äquidistante Nuten (6) vorgesehen sind.

3. Kugelkopfendoprothese nach einem der Ansprüche 1 bis 2, dadurch gekennzeichnet, daß der Kugelkopf (1) aus Metall besteht.

4. Kugelkopfendoprothese nach einem der Ansprüche 1 bis 2, dadurch gekennzeichnet, daß der Kugelkopf (1) aus Keramik besteht.

5. Total-Hüftgelenksendoprothese mit einer Kugelkopfendoprothese nach einem der Ansprüche 1 bis 4 mit einem Femurstielteil (2) und einer künstlichen Hüftgelenkspfanne mit einem Inlet aus einem hochverdichteten Polyethylen, Polyurethan und/oder Keramik als Gleitpartner für den Kugelkopf (1).

6. Total-Schultergelenksendoprothese mit einer Kugelkopfendoprothese nach den Ansprüchen 1 bis 4 mit einem Humerusstielteil und einem künstlichen Schultergelenkspfannendach mit einem Inlet aus hochverdichtetem Polyethylen, Polyurethan und/oder Keramik als Gleitpartner für den Kugelkopf (1).

## Claims

1. Ball-headed endoprosthesis consisting of a ball head corresponding to the natural joint ball which can be connected to a base part to be anchored in a bone by means of a conical clip connection, for which purpose the base part has a conical journal and the ball head a conical bore, wherein the axis (10) of the conical bore (3) encloses an angle α in the region of 7° < α < 15° with the diameter line (11) of the ball head (1), and the conical bore (3) is arranged in the ball head (1) to be decentred by a distance (V) of 1-4 mm with respect to the diameter line (11), characterised in that in the interior of the bore (3) at least five axis-parallel grooves (6) are provided, into which a locking nose (7) provided on the peripheral surface (5) of the conical journal (4) engages after producing the conical clip connection, which locking nose (7) is provided on the proximal end of the conical journal (4) pointing in the medial direction.

2. Ball-headed endoprosthesis according to claim 1, characterised in that twelve axis-parallel and equidistant grooves (6) are provided on the inner side of the conical bore (3).

3. Ball-headed endoprosthesis according to one of claims 1 to 2, characterised in that the ball head (1) consists of metal.

4. Ball-headed endoprosthesis according to one of claims 1 to 2, characterised in that the ball head (1) consists of ceramic.

5. Total hip joint endoprosthesis having a ball-headed endoprosthesis according to one of claims 1 to 4, having a femur stem part (2) and an artificial hip joint acetabulum with an inlet made from a high density polyethylene, polyurethane and/or ceramic as a sliding partner for the ball head (1).

6. Total shoulder joint endoprosthesis having a ball-headed endoprosthesis according to claims 1 to 4, having a humerus stem part and an artificial shoulder joint acetabulum roof with an inlet made from high density polyethylene, polyurethane and/or ceramic as a sliding partner for the ball head (1).

## Revendications

1. Endoprothèse d'énarthrose, constituée par une tête sphérique simulant la sphère naturelle d'articulation et qui peut être reliée au moyen d'une liaison conique de serrage à une partie de base, qui doit être ancrée dans un os, et dans laquelle à cet effet la partie de base possède un téton conique et la tête sphérique possède un perçage conique, l'axe (10) du perçage conique (3) fait avec la ligne diamétrale (11) de la tête sphérique (1) un angle α dans la gamme 7° < α < 15° et le perçage conique (3) est disposé d'une manière décentrée dans la tête sphérique (1) avec un décalage (V) de 1-4 mm par rapport à la ligne diamétrale (11), caractérisée en ce qu'à l'intérieur du perçage (3) sont prévues au moins cinq rainures (6) parallèles à l'axe, dans lesquelles s'engage, après l'établissement de la liaison conique de serrage, un bec de blocage (7) prévu sur la surface circonférentielle (5) du téton conique (4) et disposé sur l'extrémité proximale du téton conique (4) en étant dirigé dans la direction médiale.

2. Endoprothèse d'énarthrose selon la revendication 1, caractérisée en ce que douze rainures (6) parallèles à l'axe et équidistantes sont prévues dans la surface intérieure du perçage conique (3).

3. Endoprothèse d'énarthrose selon l'une des revendications 1 et 2, caractérisée en ce que la tête sphérique (1) est réalisée en un métal.

4. Endoprothèse d'énarthrose selon l'une des revendications 1 et 2, caractérisée en ce que la tête sphérique (1) est formée d'une céramique.

5. Endoprothèse totale de hanche comportant une endoprothèse d'énarthrose selon l'une des revendications 1 à 4, comportant un élément de tige fémoral (2) et une cavité artificielle de l'articulation de la hanche comportant un insert formé de polyéthylène très haute densité, de polyuréthane et/ou d'une céramique en tant que partenaire de glissement pour la tête sphérique (1).

6. Endoprothèse totale de l'articulation de l'épaule comportant une endoprothèse d'énarthrose selon les revendication 1 à 4, comportant un élément de tige huméral et un capuchon artificiel de la cavité de l'articulation de l'épaule comportant un insert formé de polyéthylène très haute densité, de polyuréthane et/ou d'une céramique en tant que partenaire de glissement pour la tête sphérique (1).
